# EUROPEAN PATENT SPECIFICATION

(11) **EP 2 352 471 B1**
(45) Date of publication and mention of the grant of the patent: **07.08.2013**
(21) Application number: 09747911.7
(22) Date of filing: 29.10.2009
(51) Int. Cl.: A61F 13/00, A61L 15/16, A61L 15/22, A61L 15/42, B32B 5/18, B32B 27/36, B32B 27/40, D04H 1/54

(54) **COMPOSITE ELASTIC MATERIAL**
ELASTISCHES VERBUNDMATERIAL
MATÉRIAU ÉLASTIQUE COMPOSITE

(30) Priority: 06.11.2008 GB 0820291
(43) Date of publication of application: 10.08.2011
(73) Proprietor: BRIGHTWAKE LIMITED, Kirkby-In-Ashfield Nottingham Nottinghamshire NG17 7JZ (GB)
(72) Inventor: COTTON, Stephen, Nottinghamshire NG15 8EQ (GB)
(74) Representative: Adamson Jones
(86) International application number: PCT/GB2009/051458
(87) International publication number: WO 2010/052485

(56) References cited:
- EP-A2- 0 425 270
- EP-A2- 1 430 911
- WO-A1-2007/046806
- WO-A1-2008/088281
- WO-A2-98/41095
- US-A- 5 540 976
- US-A- 5 865 919
- US-A- 6 074 966

## Description

This invention relates to a composite elastic material that is suitable for use as, or as a component of, a wound dressing.

Wound treatment generally involves the packing and/or covering of the wound with some form of dressing material in order to stop or prevent bleeding, absorb wound exudate, protect the wound from infection and generally provide an environment that will facilitate wound healing.

A wide variety of wound dressings are currently available. The selection of the most appropriate dressing depends on the nature and severity of the wound, as well as the environment and circumstances in which the dressing must be applied and maintained. Wound dressings associated with "first aid" situations generally consist of either cotton wool or gauze and are usually easy to apply, but function mainly to reduce initial blood loss and are only intended to remain in place temporarily. Wound dressings intended to be in place for longer periods must be fitted securely in order to effectively cover the wound and protect it from infection. Dressings consisting of standard materials tend to shift or become detached over time or during movement. Thus, considerable time and effort is required to fit a wound dressing if it is intended to remain in place for a prolonged period, particularly if the patient intends to be active. Dressings composed of gels, such as alginate and hydrocolloid are most suitable for use in an organised healthcare environment because they are awkward to prepare and apply and are not robust enough for use in situations such as when a patient needs to remain active. Current wound dressing materials are thus either adapted for use in a hospital or other prepared medical environment, or are poorly suited for prolonged application, particularly when the patient is active.

There is therefore a requirement for a dressing material that is easy to apply and that may remain securely in place for prolonged periods of time, including when the patient is active.

US 5540976 A discloses a non-woven fabric laminate having cross-stretch properties and comprising at least three layers.

WO 2008/088281 A1 discloses a wound dressing comprising a hydrophobic fabric that is to be placed towards a wound and capable of binding unwanted microorganisms, and a moisture carrying matrix such as gel or foam.

US 6074966 A discloses a non-woven fabric laminate having stretch properties, constructed using hydroentanglement methods.

EP 0425270 A2 discloses a super absorbent composite structure comprising a first porous layer, a foam-containing super absorbent second layer and a liquid impermeable third layer.

WO 98/41095 A2 discloses a multilayered antimicrobial material comprising a partly reflective base layer and a partly reflective, partly transmissive top layer.

WO 2007/046806 A1 discloses a medical device such as a wound dressing comprising a first conformable, conductive fabric comprising a tube coated with an anti-microbial metal.

EP 1430911 A2 discloses a haemostatic wound dressing containing a fabric made from biocompatible, aldehyde-modified polysaccharide fibres, and a porous polymeric matrix made from a biocompatible, water-soluble or water swellable polymer dispersed at least partially through the fabric.

US 5865919 A discloses a viral barrier comprising a thermoplastic film thermally bonded on at least one side thereof to a breathable thermoplastic web.

According to the first aspect of the invention, there is provided a composite elastic wound dressing material comprising a porous, elastic carrier layer, to one or both surfaces of which is bonded a surface layer of a non-woven fabric, wherein the surface layer is pleated when the material is relaxed such that the carrier is able to stretch.

In certain embodiments, the material according to this invention comprises a central carrier layer sandwiched between two surface layers. The two surface layers may be of similar non-woven material.

The porous elastic carrier layer may comprises relatively thin sheets of plastics foam material. Such materials may be breathable and absorbent, but may have a low tensile strength and poor resistance to abrasion. By "breathable" is meant that the material is permeable to air and moisture vapour. Other materials that may be used for the carrier layer may include stretchable plastics films, knitted fabrics and fabrics woven at least in part from elastic yarns. The material of the carrier layer may be inherently porous, or it may be perforated.

The carrier material is elastic. By this meant that the carrier material is able to stretch under the influence of an applied force without breaking, and will return substantially to its original condition upon removal of such an applied force. Typically, the carrier material exhibits elastic extension of at least 10%, or at least 20%, or at least 50% or more, in response to a force applied in the plane of the material.

The non-elastic surface layer or layers are of a non-woven fabric that may be breathable, may have a relatively high tensile strength and may be resistant to abrasion. By "non-elastic" is meant that the surface layer is not, in contrast to carrier layer, capable of substantial elastic extension.

Suitable materials for use as the carrier layer and the surface layer(s) are widely available and may be inexpensive.

One particular form of foam material that may be used as the carrier layer is thin sheet polyester foam commonly used in the packaging. One example of a suitable material is S6200 polyester foam manufactured by Caligen®.

A particular form of non-woven fabric that may be applied to the carrier layer is mechanically bonded fabric commonly used in surgical garments and masks. One example of a suitable material is Daltex® PE Spunbound material manufactured by Don & Low Ltd. Other forms of non-woven materials that may be used include spun-bonded non-wovens and melt blown materials.

The composite elastic wound dressing material according to this invention is suitable for as a wound dressing. Embodiments of the material may be elastic, breathable, soft and comfortable when in contact with the skin, resistant to abrasion and cheap and fast to manufacture. Furthermore, the surface of the dressing material consists of non-woven fabric which may act as the female component of a hook-and-loop fastening comparable to those referred to by the tradename VELCRO

The composite elastic wound dressing material according to this invention may also be absorbent, capable of being either coated or impregnated with a range of materials or agents, and may be easily sterilised. Furthermore, the composite elastic wound dressing material according to this invention may be formed using clean materials in a process that does not introduce any foreign material that may act as a potential source of contamination.

The composite elastic wound dressing material can be applied quickly and easily, either by the patient himself or an unskilled companion, and may remain in place when the patient is active.

The surface layer or layers may be bonded to the carrier layer by a number of methods.

The layers may be bonded together by adhesives. However, adhesives may solidify, reducing the elasticity, and become impermeable. Furthermore, adhesives may contain allergens and are a potential source of contamination of the material.

The layers of this composite elastic wound dressing material may be bonded by stitching. This method of joining the layers will not affect the flexibility or permeability of the material. However, in order to effectively join the multiple layers of material together, a large amount of stitching would be required, adding to the expense and time requirement of manufacture. Additionally, the stitching may have a negative effect on the elasticity of the dressing material, and the use of a large amount of a thread is a potential source of contamination of the material.

The layers of this composite elastic wound dressing material will more preferably be bonded by heat welding, which involves holding the layers of material together and heating points on the surface of the material in order to fuse the layers together. This method does not involve the introduction of any foreign elements into the material, reducing the risk of contamination. However, heat welding requires the use of high temperatures, which may damage the material of this invention. For example, the material surrounding the welding points may melt and form small irregular globules which may re-solidify into solid irregular fragments, causing irritation, discomfort or pain when the material is in contact with the skin.

The layers of this composite elastic wound dressing material will most preferably be bonded by ultrasonic welding. The process of ultrasonic welding involves the application of high-frequency acoustic vibrations to materials held together under pressure, producing sufficient levels of localised friction and heat to weld the materials together. This process is generally used to weld thermoplastic components or fabrics made at least in part from synthetic fibres. Ultrasonic welding is a particularly desirable technique for producing materials for use in the medical industry, such as surgical garments and masks, because it does not introduce any potential contaminants, such as adhesives, into the material. Other advantages of this technique include rapidity and high controllability in comparison to equivalent techniques such as heat welding, since it can be halted instantaneously without any residual effect, such as heat, and be restricted to a very limited part of the material without affecting the surrounding regions.

Ultrasonic welding machines generally consist of three main components: a converter which uses disks of piezoelectric material to convert electrical energy into high frequency vibrations, an amplitude modifier (referred to as a booster) which increases the amplitude of the vibrations, and a sonotrode (referred to as a horn) which transmits the vibrations to the material. Welding is carried out by applying vibrations to material held under pressure between the sonotrode and a holding surface (referred to as an anvil).

In one embodiment of a manufacturing process for a composite elastic wound dressing-material according to the invention, the layers of material that make up the composite material are fed to a roller that has a multitude of regularly spaced flat-tipped pin-like projections on its surface. This so-called pin-roller and the sonotrode are configured so when the pin-roller is rotated, the tips of the pins pass close to the sonotrode surface. In operation, the layers of material are fed between the sonotrode and the pin roller, and the tips of the pins act as points of increased pressure between the pin-roller and the sonotrode where welding can occur. In this way, the tips of the pins act as a multitude of individual anvils. The separate layers of material are bonded together at the points where the tips of the pins squeeze the layers together. The use of the pin-roller allows ultrasonic welding to be carried out as a continuous process and, because only the regions of the material that contact the tips of the pins are welded, the properties of the remaining material are preserved.

The pin-roller and sonotrode may be set to provide differing degrees of pressure between the tips of the pins and the sonotrode surface. This may result in the variation in the strength of the welds, or cause the pins to pierce the material fully, producing a multitude of small, regularly spaced apertures in the material.

The required elasticity is conferred on the composite elastic wound dressing material according to this invention by binding the elastic carrier layer to the non-elastic surface layer or layers when the elastic carrier layer is under tension and in a stretched conformation. Thus, when the tension is released, the elastic carrier layer relaxes and the adjoined non-elastic surface layers crumple, providing sufficient slack in the non-elastic outer layers to allow the material to be returned to the stretched conformation. The crumpling of the outer layers of the material also adds to the thickness of the material, imparting to it further cushioning properties and enhancing the soft feel of the material.

The thickness of the carrier layer can be increased in order to enhance the absorbance capacity and cushioning properties of the material. The carrier layer may have a thickness ranging from 0.2mm to about 10mm, more typically ranging from 0.5mm to 5mm and yet more typically ranging from 1 mm to 2mm.

In embodiments of this invention, the composite elastic wound dressing material is fully permeable to air and may therefore be placed in contact with the skin without causing irritation or discomfort. The material may also be permeable to fluids such as wound exudate, and may therefore assist in the transport of such fluids away from the wound site.

The structure of non-woven material is such that the fibres of the fabric are irregularly arranged and form a tangled mat. Thus, the surface of the composite material formed of non-woven fabric may be effectively engaged by a surface carrying a multitude of hook components and thereby act as the loop surface in a hook-and-loop fastening arrangement.

The composite elastic wound dressing material according to this invention will most preferably be sterile so as not to introduce infective agents into the wound. Where the materials used are sensitive to heat, sterilisation methods using heat or pressure are not suitable. A more preferred method of sterilisation may be gamma irradiation or chemical sterilisation using agents such as ethylene oxide, both of which are commonly used for the sterilisation of medical equipment.

Typically, the composite elastic wound dressing material according to the invention may be manufactured in elongated form, and may be wound onto a roll for supply to an end user or for storage as an intermediate product prior to use of the material in the manufacture of a finished product.

In many embodiments, the composite elastic wound dressing material according to this invention is elastic only along its longitudinal axis, ie the axis along which the elastic carrier layer is stretched during manufacture of the composite material, when the carrier layer is bonded to the non-elastic surface layer(s). However, for some applications (eg when dressing a wound on or near a joint) it may also be necessary or desirable for the material to also be elastic along its transverse axis. To achieve this, multiple slits may be formed in the material, aligned parallel to the longitudinal axis. Such slits will allow for some expansion of the material along the transverse axis as required.

The composite elastic wound dressing material according to the invention may be covered on at least one side by a non-adherent layer, such as a hydrophobic gel or synthetic plastic film, to prevent adherence to a wound surface while in place. The adherence of a dressing to a wound is undesirable because it causes difficulties in redressing, as well as pain and trauma for the patient. In order for fluid to be transmitted away from the wound, the non-adherent layer may also be perforated to allow free passage of fluid.

In order to facilitate rapid application of the dressing, the material according to the invention may be coated on one or both sides with a contact adhesive.

The composite elastic wound dressing material according to this invention may also be covered on one side with an impermeable film which can act as barrier to prevent entry of hazardous agents. This may be of particular utility to the military or emergency services by providing a wound dressing that is able to protect wounds from any hazardous agents that may be present in the surrounding environment, such as dangerous chemical and biological agents.

The composite elastic wound dressing material according to the invention may be coated on a wound-contacting side thereof with a layer of water soluble polymer material (eg polyvinyl alcohol) impregnated with a blood clotting agent. When fluid from the wound makes contact with the dressing, the polymer material will begin to dissolve, releasing the clotting agent into the wound. This may be of particular utility as a dressing for application to a wound immediately after it has been sustained (ie a trauma dressing) as it provides a dressing which, when activated by the presence of a fluid such as blood, will release a blood clotting agent which may serve to reduce initial blood loss.

The invention will now be described in greater detail, by way of example only, with reference to the accompanying drawings, in which
Figure 1 is a plan view of a length of a first embodiment of a composite material according to this invention;
Figure 2A is a schematic cross-sectional view of the material of Figure 1 in a relaxed conformation;
Figure 2B is a view similar to Figure 2A, but showing the material in a stretched conformation;
Figure 3 is a view similar to Figure 1 of a second embodiment of a composite material according to the invention that is pierced by multiple slits that extend along the longitudinal axis of the material; and
Figure 4 is a schematic representation of a process by which the material of Figures 1 to 3 may be manufactured.

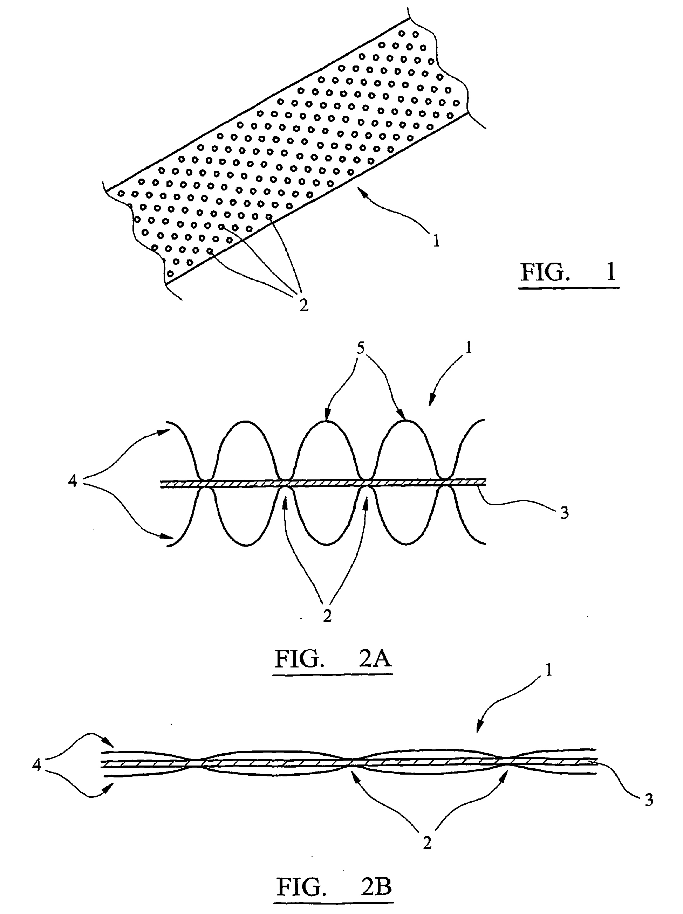

Referring first to Figure 1, a first embodiment of a composite material according to the invention is generally designated 1. On the surface of the material there are indicated a multitude of regularly spaced points 2 at which the layers of the material 1 (described below) are joined by ultrasonic welding. Depending on the conditions used in the ultrasonic welding process, the material 1 may be punctured at these weld points 2, which therefore define perforations in the material 1.

Figure 2A is a schematic cross-sectional view of the material 1, in a relaxed conformation. The material 1 comprises a central elastic layer 3 consisting of thin sheet polyester foam with a thickness of from about 0.5mm to 2mm, and two outer non-elastic layers 4 consisting of mechanically bonded non-woven fabric with a thickness of about 0.1 mm. The central layer 3 and surface layers 4 are joined at the weld points 2.

The elastic central layer 3 is bonded to the non-elastic surface layers 4 when in a stretched conformation, so that when it returns to its relaxed conformation, the adjoined surface layers slacken and form a multitude of pleats 5. The pleats 5 in the non-elastic surface layers 4 allow the central elastic layer 3 to stretch without being restricted by the non-elastic surface layers 4 up to the point where they become taut.

Figure 2B is a view similar to Figure 2A, but showing the material 1 in a stretched conformation. The pleats 5 in the outer non-elastic layers 4 are no longer present as the outer layers are now taut.

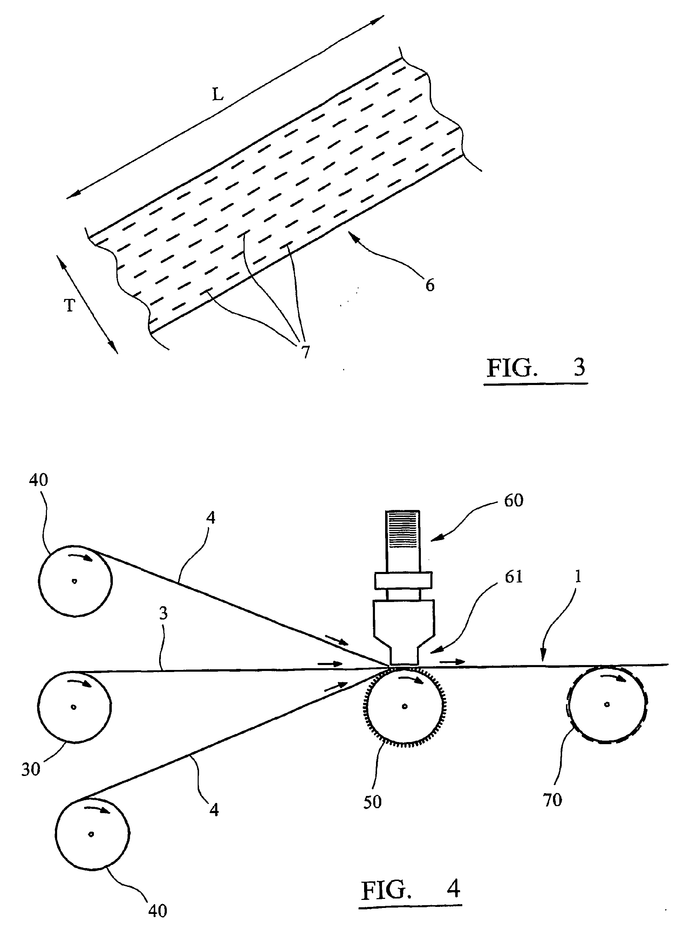

Referring now to Figure 3, a second embodiment of the composite material according to the invention is generally designated 6. In this embodiment, the composite material is pierced by a multitude of slits 7 that are aligned along the longitudinal axis of the material. The way in which the slits are arranged allows the material to stretch along the transverse axis T, while the arrangement of the composite material allows it to stretch along the longitudinal axis L. Apart from the provision of slits 7, the second embodiment 6, is similar to the first embodiment 1. The multitude of regularly spaced weld points is not shown in Figure 3 for the sake of clarity.

Referring now to Figure 4, there is depicted an apparatus for the manufacture of the composite material as described in Figures 1 to 3. This apparatus consists of an ultrasonic welding assembly generally designated 60 having a sonotrode 61 which may transmit vibrations to a material, a pin roller 50 having a multitude of regularly spaced flat-tipped pin-like projections on its surface and positioned relative to the sonotrode so that when rotated the tips of the pins pass close to the sonotrode surface, a number of rollers 30, 40 positioned upstream of the pin-roller for discharging layers of material 3, 4 to the pin-roller 50, and an optional cutting roller 70 positioned downstream of the pin-roller 50 and having a multitude of regularly spaced, thin, elongated projections that form cutting blades.

In operation, the elastic central material 3 is discharged from roller 30 and the two layers of non-elastic surface material 4 are discharged from rollers 40 on either side of the elastic central material 3, causing the elastic central layer 3 to be sandwiched between the two non-elastic surface layers 4 as they are fed to the pin-roller 50. The rotation of the pin-roller 50 draws the layers of material 3, 4 between the pin-roller 50 and the sonotrode 61. At the point where the pin roller 50 is closest to the sonotrode surface, the pins act as points of compression between the pin-roller 50 and the sonotrode 61, where the layers of material 3, 4 are welded together to form composite material 1 depicted in Figure 1.

The position of the pin-roller 50 in relation to the sonotrode 61 may be adjusted in order to vary the pressure between the tips of the pins and the sonotrode surface when the apparatus is in operation. This may result in the variation of the strength of welding and potentially produce perforations in the material at the weld points 2.

The rate at which the non-elastic surface layers 4 are discharged by rollers 40 is equal to the rate at which they are engaged by the pin-roller 50. The rate at which the elastic central layer 3 is discharged by roller 30, is slower than the rate at which it is engaged by the pin-roller 50 which places the elastic central layer 3 under tension, causing it to be stretched as it is engaged by the pin-roller 50 and welded to the non-elastic surface layers 4. Thus when the tension is removed from the composite material, the elastic central layer 3 returns to its native conformation, causing the adjoined non-elastic surface layers 4 to slacken and form the pleats 5 depicted in Figure 2A.

The cutting-roller 70 may optionally be positioned downstream of the pin-roller in order to produce composite material 6 as depicted in Figure 3. The multitude of cutting blades on the surface of the cutting roller 70 may pierce the material as it is fed onto the cutting roller, producing the slits 7 depicted in Figure 3.

## Claims

1. A composite elastic wound dressing material comprising a porous, elastic carrier layer, to one or both surfaces of which is bonded a non-elastic surface layer of a non-woven fabric, wherein the surface layer is pleated when the material is relaxed such that the carrier layer is able to stretch.

2. A composite elastic material as claimed in Claim 1, wherein a surface layer is bonded to both surfaces of the carrier layer.

3. A composite elastic material as claimed in any preceding claim, wherein the carrier layer is a sheet of plastics foam.

4. A composite elastic material as claimed in Claim 3, wherein the carrier layer is a sheet of polyester foam.

5. A composite elastic material as claimed in any preceding claim, wherein the carrier layer has a thickness of between 0.2mm and 10mm, or between 0.5mm and 5mm, or between 1 mm and 2mm.

6. A composite elastic material as claimed in any preceding claim, wherein the layers of the material are bonded by ultrasonic welding.

7. A composite elastic material as claimed in any preceding claim, wherein the material is provided in elongated form.

8. A composite elastic material as claimed in Claim 7, wherein the material is provided with multiple slits aligned parallel to the longitudinal axis of the material to allow for expansion of the material along its transverse axis.

9. A composite elastic material as claimed in any preceding claim, wherein material is covered on one side with a layer of non-adherent material.

10. A composite elastic material as claimed in any one of Claims 1 to 8, wherein the material is coated on one or both sides with a contact adhesive.

11. A composite elastic material as claimed in any preceding claim, wherein the surface layer or layers act as the female component of a hook-and-loop fastening.

12. A composite elastic material as claimed in any preceding claim, wherein the material forms a skin-contacting part of a medical product.

13. A method of manufacturing a composite elastic wound dressing material as claimed in Claim 1, which method comprises the steps of:
a) providing an apparatus comprising a roller having a multitude of regularly spaced flat-tipped pin-like projections on its surface (pin-roller), and a sonotrode that is configured relative to the pin-roller such that, when the pin-roller is rotated, the tips of the pins pass close to the sonotrode surface;
b) feeding a layered assembly, comprising a carrier layer and a surface layer, onto the pin-roller as it is rotated; and
c) applying high-frequency mechanical vibrations from the sonotrode to the layered assembly, such that the layered assembly is bonded together at the points at which it contacts the pins,
wherein the carrier layer is in a stretched conformation when the surface layer is bonded to it.

14. A method as claimed in Claim 13, wherein the pin-roller and sonotrode are configured such that the pins fully pierce the layered assembly.

15. A method as claimed in Claim 13 or 14, wherein the composite elastic material is produced in elongated form.

## Patentansprüche

1. Elastisches Verbund-Wundverbandmaterial, das eine poröse, elastische Trägerlage umfasst, auf deren eine oder beide Flächen eine nichtelastische Oberflächenlage aus einem Vliesstoff geheftet ist, wobei die Oberflächenlage im entspannten Zustand des Materials gefaltet wird, so dass sich die Trägerlage dehnen kann.

2. Elastisches Verbundmaterial nach Anspruch 1, wobei die Oberflächenlage auf beide Flächen der Trägerlage geheftet ist.

3. Elastisches Verbundmaterial nach einem der vorherigen Ansprüche, wobei die Trägerlage eine geschäumte Kunststofffolie ist.

4. Elastisches Verbundmaterial nach Anspruch 3, wobei die Trägerlage eine geschäumte Polyesterfolie ist.

5. Elastisches Verbundmaterial nach einem der vorherigen Ansprüche, wobei die Trägerlage eine Dicke zwischen 0,2 mm und 10 mm oder zwischen 0,5 mm und 5 mm oder zwischen 1 mm und 2 mm hat.

6. Elastisches Verbundmaterial nach einem der vorherigen Ansprüche, wobei die Lagen des Materials durch Ultraschallschweißen aufgeklebt sind.

7. Elastisches Verbundmaterial nach einem der vorherigen Ansprüche, wobei das Material in länglicher Form bereitgestellt wird.

8. Elastisches Verbundmaterial nach Anspruch 7, wobei das Material mit mehreren Schlitzen versehen ist, die parallel zur Längsachse des Materials ausgerichtet sind, um eine Expansion des Materials entlang seiner Querachse zuzulassen.

9. Elastisches Verbundmaterial nach einem der vorherigen Ansprüche, wobei Material auf einer Seite mit einer Schicht aus nicht adhärentem Material bedeckt ist.

10. Elastisches Verbundmaterial nach einem der Ansprüche 1 bis 8, wobei das Material auf einer oder auf beiden Seiten mit einem Kontaktklebstoff beschichtet ist.

11. Elastisches Verbundmaterial nach einem der vorherigen Ansprüche, wobei die Oberflächenschicht(en) als weibliche Komponente eines Klettverschlusses wirkt/-en.

12. Elastisches Verbundmaterial nach einem der vorherigen Ansprüche, wobei das Material einen Hautkontaktteil eines medizinischen Produkts bildet.

13. Verfahren zur Herstellung eines elastischen Verbund-Verbandmaterials nach Anspruch 1, wobei das Verfahren die folgenden Schritte beinhaltet:
a) Bereitstellen einer Vorrichtung, die eine Rolle mit mehreren gleichmäßig beabstandeten stiftähnlichen Vorsprüngen mit flacher Spitze auf ihrer Oberfläche (Stiftrolle) und eine Sonotrode aufweist, die relativ zur Stiftrolle so konfiguriert ist, dass die Spitzen der Stifte beim Drehen der Stiftrolle nahe an der Sonotrodenfläche passieren;
b) Zuführen einer mehrlagigen Baugruppe, die eine Trägerschicht und eine Oberflächenschicht umfasst, auf die Stiftrolle, während diese rotiert; und
c) Aufbringen von mechanischen Hochfrequenzvibrationen von der Sonotrode auf die mehrlagige Baugruppe, so dass die mehrlagige Baugruppe an den Punkten, an denen sie mit den Stiften in Kontakt kommt, zusammengeheftet wird,
wobei die Trägerschicht in einer gedehnten Konformation ist, wenn die Oberflächenlage darauf geklebt wird.

14. Verfahren nach Anspruch 13, wobei die Stiftrolle und die Sonotrode so konfiguriert sind, dass die Stifte die mehrlagige Baugruppe vollständig durchbohren.

15. Verfahren nach Anspruch 13 oder 14, wobei das elastische Verbundmaterial in länglicher Form erzeugt wird.

## Revendications

1. Matériau pour pansement élastique composite, comprenant une couche de support poreuse, élastique, sur l'une ou les deux surfaces de laquelle est liée une couche de surface non élastique d'un tissu non tissé, où la couche de surface est plissée lorsque le matériau est détendu de telle sorte que la couche de support soit en mesure de s'étirer.

2. Matériau composite élastique tel que revendiqué dans la Revendication 1, où une couche de surface est liée aux deux surfaces de la couche de support.

3. Matériau composite élastique tel que revendiqué dans une quelconque revendication précédente, où la couche de support est une feuille de mousse en matière plastique.

4. Matériau composite élastique tel que revendiqué dans la Revendication 3, où la couche de support est une feuille de mousse de polyester.

5. Matériau composite élastique tel que revendiqué dans une quelconque revendication précédente, où la couche de support a une épaisseur comprise entre 0,2 mm et 10 mm, ou entre 0,5 mm et 5 mm, ou entre 1 mm et 2 mm.

6. Matériau composite élastique tel que revendiqué dans une quelconque revendication précédente, où les couches du matériau sont liées par soudage par ultrasons.

7. Matériau composite élastique tel que revendiqué dans une quelconque revendication précédente, où le matériau est fourni sous forme allongée.

8. Matériau composite élastique tel que revendiqué dans la Revendication 7, où le matériau est pourvu de plusieurs fentes alignées en parallèle à l'axe longitudinal du matériau pour permettre l'expansion du matériau le long de son axe transversal.

9. Matériau composite élastique tel que revendiqué dans une quelconque revendication précédente, où le matériau est recouvert sur un côté d'une couche de matériau non adhérent.

10. Matériau composite élastique tel que revendiqué dans une quelconque des Revendications 1 à 8, où le matériau est revêtu sur un ou les deux côtés d'un adhésif de contact.

11. Matériau composite élastique tel que revendiqué dans une quelconque revendication précédente, où la couche ou les couches de surface agissent en tant que composant femelle d'une fermeture à crochet et boucle.

12. Matériau composite élastique tel que revendiqué dans une quelconque revendication précédente, où le matériau forme une partie de contact avec la peau d'un produit médical.

13. Procédé de fabrication d'un matériau pour pansement composite élastique tel que revendiqué dans la Revendication 1, lequel procédé comprend les étapes consistant à :
a) fournir un appareil comprenant un cylindre ayant une multitude de saillies semblables à des pointes à bout plat espacées régulièrement sur sa surface (cylindre à pointes), et une sonotrode qui est configurée par rapport au cylindre à pointes de telle sorte que, lorsque le cylindre à pointes est mis en rotation, les bouts des pointes passent à proximité de la surface de la sonotrode ;
b) introduire un ensemble en couches, comprenant une couche de support et une couche de surface, sur le cylindre à pointes tandis qu'il est mis en rotation ; et
c) appliquer des vibrations mécaniques à haute fréquence de la sonotrode vers l'ensemble en couches, de telle sorte que l'ensemble en couches soit lié au niveau des points où il est en contact les pointes,
où la couche de support est dans une conformation étirée lorsque la couche de surface est liée à celle-ci.

14. Procédé tel que revendiqué dans la Revendication 13, où le cylindre à pointes et la sonotrode sont configurés de telle sorte que les pointes percent entièrement l'ensemble en couches.

15. Procédé tel que revendiqué dans la revendication 13 ou 14, où le matériau composite élastique est produit sous forme allongée.
